# EUROPÄISCHE PATENTSCHRIFT

(11) **EP 0 505 400 B1**
(45) Veröffentlichungstag und Bekanntmachung des Hinweises auf die Patenterteilung: **10.03.1999**
(21) Anmeldenummer: 91900221.2
(22) Anmeldetag: 13.12.1990
(51) Int. Cl.: C07D 221/14, A61K 31/47

(54) **N-SUBSTITUIERTE NAPHTHALIMIDE, IHRE HERSTELLUNG UND VERWENDUNG**
N-SUBSTITUTED NAPHTHALAMIDES, THEIR PRODUCTION AND USE
NAPHTALIMIDES N-SUBSTITUES, LEUR FABRICATION ET UTILISATION

(30) Priorität: 21.12.1989 DE 3942280
(43) Veröffentlichungstag der Anmeldung: 30.09.1992
(73) Patentinhaber: KNOLL AG, D-67061 Ludwigshafen (DE)
(72) Erfinder: FERNANDEZ BRANA, Miguel, E-28036 Madrid (ES); KLUGE, Michael, D-6701 Kallstadt (DE); KEILHAUER, Gerhard, D-6701 Dannstadt-Schauernheim (DE); MORAN MOSET, Marina, E-28015 Madrid (ES); CASTELLANO BERLANGA, Jose, Maria, E-28036 Madrid (ES)
(74) Vertreter: Karau, Wolfgang, Dr.
(86) Internationale Anmeldenummer: EP9002163
(87) Internationale Veröffentlichungsnummer: WO9109850

(56) Entgegenhaltungen:
- EP-A- 0 125 439
- EP-A- 0 268 093
- FR-A- 2 392 978
- US-A- 4 146 720

## Beschreibung

Die Erfindung betrifft neue N-substituierte Naphthalimide, ein Verfahren zu deren Herstellung sowie deren Verwendung bei der Bekämpfung von Krankheiten.

Es ist bereits bekannt, daß bestimmte Benzo[de]isochinoline tumorhemmende Eigenschaften besitzen (Afinidad 35, 105 (1978), Cancer Chemother. Pharmacol. 4, 61 (1980), Eur. J. Med. Chem.-Chimica Ther. 16, 207 (1981), Arzneim. Forsch./Drug Research 34 (II), 1243 (1984), J. Med. Chem. 28, 1216 (1985), EP-A-0 268 093, FR-A-2 392 978, EP-A-0 125 439, US-A-4 146 720). Die Wirkungen dieser Verbindungen sind jedoch nicht in jeder Hinsicht befriedigend.

Es wurde nun gefunden, daß
a. 3-Amino-N-(2-methyl-2-dimethylaminoethyl)-1,8-naphthalimid,
b. 3-Amino-N-(1-methyl-2-dimethylaminoethyl)-1,8-naphthalimid,
c. 3-Amino-N-(2,2-dimethyl-2-dimethylaminoethyl)-1,8-naphthalimid,
d. 3-Amino-N-(2-diethylamino-2-methylethyl)-1,8-naphthalimid,
e. 3-Amino-N-[2-methyl-2-(1-pyrrolidinyl)ethyl]-1,8-naphthalimid, und
f. 3-Amino-N-[2-methyl-2-(1-piperidinyl)ethyl]-1,8-naphthalimid
sowie deren Salze mit physiologisch verträglichen Säuren, eine bessere Wirkung bzw. ein besseres Wirkungsspektrum als tumorhemmende Substanzen besitzen und antileukämisch wirken.

Als physiologisch verträgliche Säuren eignen sich zur Salzbildung organische und anorganische Säuren, wie Salzsäure, Schwefelsäure, Phosphorsäure, Essigsäure, Zitronensäure, Oxalsäure, Malonsäure, Salizylsäure, Maleinsäure, Fumarsäure, Bernsteinsäure, Ascorbinsäure, Äpfelsäure, Methansulfonsäure, Isethionsäure, Milchsäure, Gluconsäure, Glucuronsäure, Amidosulfonsäure, Benzoesäure, Weinsäure, Pamoasäure.

Die neuen Verbindungen können in solvatisierter Form vorliegen. Solche Formen können sich beispielsweise mit Wasser oder Ethanol bilden.

Die neuen Verbindungen werden hergestellt, indem man ein 3-Amino-1,8-naphthalsäureanhydrid mit dem entsprechenden Diamin und die so erhaltenen Verbindungen gegebenenfalls in deren Salze mit physiologisch verträglichen Säuren überführt.

Die Umsetzung wird in einem geeigneten Lösungsmittel wie Dimethylformamid, Methanol, Ethanol, Propanol oder Aceton in der Regel bei Raumtemperatur durchgeführt. Die erfindungsgemäße Verbindung scheidet sich aus dem Reaktionsgemisch ab und kann chromatographisch und/oder durch Umkristallisation gereinigt werden.

Die so erhaltenen Verbindungen können in an sich bekannter Weise in ihre Salze überführt werden, z.B. durch Umsetzen mit einer Säure.

Die Ausgangsverbindungen für die Herstellung der neuen Verbindungen sind bekannt oder lassen sich nach bekannten Verfahren herstellen.

Die erfindungsgemäßen Verbindungen können in üblicher Weise oral oder parenteral verabfolgt werden. Sie können in den gebräuchlichen galenischen Applikationsformen fest oder flüssig angewendet werden, z.B. als Tabletten, Filmtabletten, Kapseln, Granulate, Dragees oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 10 bis 90 Gew.-%.

Die neuen Verbindungen wurden analog folgender Vorschrift hergestellt:

Eine Suspension von 4,3 g (20 mMol) 3-Nitro-1,8-naphthalsäureanhydrid und 2,1 g (20 mMol) 2-Dimethylamino-l-propylamin in 60 ml Ethanol wurde bei Raumtemperatur 24 h gerührt. Der Niederschlag wurde abfiltriert und aus Ethanol umkristallisiert. Es wurden 4,2 g (70 % N-(2-Methyl-2-dimethylaminoethyl)-3-nitro-1,8-naphthalimid (F = 195°C) erhalten.

Es wurden erhalten:
1. 3-Amino-N-(2-methyl-2-dimethylaminoethyl)-1,8-naphthalimid, F. 195°C (Methanol),
2. 3-Amino-N-(1-methyl-2-dimethylaminoethyl)-1,8-naphthalimid, F. 170°C (Toluol),
3. 3-Amino-N-(2,2-dimethyl-2-dimethylaminoethyl)-1,8-naphthalimid, F. 220°C (Toluol),
4. 3-Amino-N-(2-diethylamino-2-methylethyl)-1,8-naphthalimid, F. 183°C (Ethanol),
5. 3-Amino-N-[2-methyl-2-(1-pyrrolidinyl)ethyl]-1,8-naphthalimid, F. 169°C (Toluol),
6. 3-Amino-N-[2-methyl-2-(1-piperidinyl)ethyl]-1,8-naphthalimid, F. 135°C (Ethanol).

## Patentansprüche

1. N-substituiertes Naphthalimid ausgewählt aus der Gruppe bestehend aus
a. 3-Amino-N-(2-methyl-2-dimethylaminoethyl)-1,8-naphthalimid,
b. 3-Amino-N-(1-methyl-2-dimethylaminoethyl)-1,8-naphthalimid,
c. 3-Amino-N-(2,2-dimethyl-2-dimethylaminoethyl)-1,8-naphthalimid,
d. 3-Amino-N-(2-diethylamino-2-methylethyl)-1,8-naphthalimid,
e. 3-Amino-N-[2-methyl-2-(1-pyrrolidinyl)ethyl]-1,8-naphthalimid, und
f. 3-Amino-N-[2-methyl-2-(1-piperidinyl)ethyl]-1,8-naphthalimid sowie deren Salze mit physiologisch verträglichen Säuren.

2. Verfahren zur Herstellung der N-substituierten Naphthalimide gemäß Anspruch 1, dadurch gekennzeichnet, daß man 3-Amino-1,8-naphthalsäureanhydrid mit dem entsprechenden Diamin umsetzt und die so erhaltenen Verbindungen gegebenenfalls in deren Salze mit physiologisch verträglichen Säuren überführt.

3. N-substituierte Naphthalimide gemäß Anspruch 1 zur Verwendung bei der Bekämpfung von Krankheiten.

4. Verwendung der N-substituierten Naphthalimide gemäß Anspruch 1 zur Herstellung von Arzneimitteln, die das Tumorwachstum hemmen und antileukämisch wirken.

## Claims

1. N-Substituted naphthalimide selected from the group consisting of
a. 3-Amino-N-(2-methyl-2-dimethylaminoethyl)-1,8-naphthalimide,
b. 3-Amino-N-(1-methyl-2-dimethylaminoethyl)-1,8-naphthalimide,
c. 3-Amino-N-(2,2-dimethyl-2-dimethylaminoethyl)-1,8-naphthalimide,
d. 3-Amino-N-(2-diethylamino-2-methylethyl)-1,8-naphthalimide,
e. 3-Amino-N-[2-methyl-2-(1-pyrrolidinyl)ethyl]-1,8-naphthalimide,
f. 3-Amino-N-[2-methyl-(1-piperidinyl)ethyl]-1,8-naphthalimide,
and the salts thereof with physiologically tolerated acids.

2. Process for the preparation of the N-substituted naphthalimides according to claim 1, characterised in that 3-amino-1,8-naphthalic anhydride is reacted with the appropriate diamine and the resulting compounds are converted into their salts with physiologically tolerated acids where appropriate.

3. N-Substituted naphthalimides according to claim 1 for use for controlling diseases.

4. Use of the N-substituted naphthalimides according to claim 1 for the preparation of drugs which inhibit tumor growth and have antileukemic actions.

## Revendications

1. Naphtalimides substitués à l'azote, choisis dans le groupe consistant en
a. le 3-amino-N-(2-méthyl-2-diméthylaminoéthyl)-1,8-naphtalimide,
b. le 3-amino-N-(1-méthyl-2-diméthylaminoéthyl)-1,8-naphtalimide,
c. le 3-amino-N-(2,2-diméthyl-2-diméthylaminoéthyl)-1,8-naphtalimide,
d. le 3-amino-N-(2-diéthylamino-2-méthyléthyl)-1,8-naphtalimide,
e. le 3-amino-N-[2-méthyl-2-(1-pyrrolidinyl)éthyl]-1,8-naphtalimide
et
f. le 3-amino-N-[2-méthyl-2-(1-pipéridinyl)éthyl]-1,8-naphtalimide
et leurs sels d'acides acceptables pour l'usage pharmaceutique.

2. Procédé de préparation des naphtalimides substitués à l'azote selon la revendication 1, caractérisé par le fait que l'on fait réagir l'anhydride 3-amino-1,8-naphtalique avec la diamine correspondante et le cas échéant on convertit les composés obtenus en leurs sels d'acides acceptables pour l'usage pharmaceutique.

3. Les naphtalimides substitués à l'azote selon la revendication 1, pour l'utilisation dans le traitement de maladies.

4. Utilisation des naphtalimides substitués à l'azote selon la revendication 1, pour la préparation de médicaments inhibiteurs de la croissance des tumeurs et antileucémiques.
